# EUROPEAN PATENT SPECIFICATION

(11) **EP 4 368 204 B1**
(45) Date of publication and mention of the grant of the patent: **13.08.2025**
(21) Application number: 22877976.5
(22) Date of filing: 09.10.2022
(51) Int. Cl.: A61K 35/74, A61K 39/395, C07K 16/28, A61P 35/00

(54) **USE OF SPORE OF CLOSTRIDIUM GHONII IN COMBINATION WITH PEMBROLIZUMAB**
VERWENDUNG VON SPOREN VON CLOSTRIDIUM GHONII IN KOMBINATION MIT PEMBROLIZUMAB
UTILISATION DE SPORES DE CLOSTRIDIUM GHONII EN COMBINAISON AVEC DU PEMBROLIZUMAB

(30) Priority: 09.10.2021 CN 202111177854
(43) Date of publication of application: 15.05.2024
(73) Proprietor: Shihuida Pharmaceutical Group (Jilin) Co., Ltd., Baishan Jilin 134399 (CN)
(72) Inventor: WANG, Yong, Jinan, Shandong 250101 (CN); ZHU, Hong, Jinan, Shandong 250101 (CN); ZHANG, Wenhua, Jinan, Shandong 250101 (CN); XING, Yanqiu, Jinan, Shandong 250101 (CN); WANG, Dan, Jinan, Shandong 250101 (CN); LIU, Yuanyuan, Jinan, Shandong 250101 (CN); WANG, Shaopeng, Jinan, Shandong 250101 (CN); ZHENG, Jiahui, Jinan, Shandong 250101 (CN); ZHANG, Rong, Jinan, Shandong 250101 (CN); LI, Xiaonan, Jinan, Shandong 250101 (CN); XU, Xinglu, Jinan, Shandong 250101 (CN); JIANG, Shengbiao, Jinan, Shandong 250101 (CN); XING, Lichao, Jinan, Shandong 250101 (CN); GAO, Yuxia, Jinan, Shandong 250101 (CN); SHAO, Shili, Jinan, Shandong 250101 (CN); HAN, Ting, Jinan, Shandong 250101 (CN)
(74) Representative: Moré, Solveig Helga
(86) International application number: PCT/CN2022/124020
(87) International publication number: WO 2023/056962

(56) References cited:
- WO-A1-2017/210637
- CN-A- 106 265 760
- CN-A- 108 102 945
- CN-A- 109 771 445
- CN-A- 111 629 739
- CN-A- 113 995 835
- US-A1- 2021 000 885
- US-B2- 9 962 412

## Description

The present application claims priority to the Chinese Patent Application No. CN202111177854.3, filed with the China National Intellectual Property Administration (CNIPA) on October 09, 2021, and entitled "USE OF *CLOSTRIDIUM GHONII* SPORE COMBINED WITH PD-1 ANTIBODY'.

### TECHNICAL FIELD

The present disclosure belongs to the technical field of biomedicine, and in particular relates to use of *Clostridium ghonii* combined with pembrolizumab.

### BACKGROUND

Tumor microenvironment (TME) is a local steady-state environment composed of tumor cells, stromal cells (including fibroblasts, immune and inflammatory cells, and some vascular endothelial cells, etc.), extracellular matrix and biomolecules infiltrated therein during a tumor growth process. The TME provides necessary material basis for tumorigenesis, development, invasion, etc., and regulates and controls biological behaviors such as tumor metastasis and relapse. Meanwhile, the TME can increase drug resistance and radiation resistance of tumors and reduces a treatment effect. Immunomodulation in the TME has an important function in tumorigenesis and development and leads to local immunosuppression in tumors through a variety of mechanisms. Therefore, how to regulate a TME-based immunotherapy strategy and remodel an active immune microenvironment are important for an anti-tumor therapy.

Pembrolizumab is a PD-1 inhibitor. PD-1 is an important immunosuppressive molecule, thus a PD-1 antibody shows a significant anti-tumor effect clinically, including a lasting therapeutic effect on a patient in a late stage metastasis. WO2017/210637 discloses an anti-PD-1 antibody, in particular nivolumab or pembrolizumab, for use in the treatment of colon cancer. It is generally accepted that sensitivity to immune checkpoint blockers depends on tumor neoantigen burden and an infiltration degree and a composition of immune cells in the TME. Unfortunately, most common cancers do not exhibit extensive mutation and immune cell infiltration. Thus, tumors are insensitive to the immune checkpoint inhibitors with a low response rate and the immune checkpoint inhibitors are effective to only 20% of patients. Therefore, it is also one of major research directions at present to develop methods enabling the tumors to be more sensitive to immunotherapy.

*Clostridium ghonii* is a strict anaerobe, has a tumor-oriented hypoxic property, and can only colonize in a tumor hypoxia area. Oncolysis by the *Clostridium ghonii* can recruit a large number of immune cells to infiltrate into the TME, including innate immune cells of dendritic cells, neutrophils, and macrophages, specific immune cells of CD³⁺ T, CD⁴⁺ T, and CD⁸⁺ T cells, and thus changes the infiltration degree and the composition of the immune cells in the TME. At the same time, the oncolysis by the *Clostridium ghonii* enhances expressions of cytokines and chemokines such as TNF-α, IFN-γ, and IL-6 in tumors. It can be seen that oncolysis by *Clostridium ghonii* can affect immunogenicity of a tumor microenvironment (TME) by various ways, converts an immunosuppressive state of the TME into an immune-activated state, adjusts the immunosuppressive TME, and breaks an immune tolerance. US9962412 discloses that *Clostridium ghonii,* or spores thereof, is effective in treating colon cancer; the *Clostridium ghonii* can be the strain with deposit number V12/001485, V12/001486 or V12/001487.

Therefore, it is possible to effectively prevent tumors by the *Clostridium ghonii* combined with the immune checkpoint inhibitor.

### SUMMARY

According to deficiencies of the prior art, the present disclosure provides use of a *Clostridium ghonii* spore combined with a PD-1 antibody.

The invention is defined in the claims.

The present disclosure adopts the following technical solutions.

Use of a *Clostridium ghonii* spore combined with pembrolizumab in preparing a pharmaceutical product for treating colon cancer is provided.

A drug for treating colon cancer is provided, where the drug includes active ingredients of a *Clostridium ghonii* spore and pembrolizumab.

According the present disclosure, preferably, the *Clostridium ghonii* may be a *Clostridium ghonii* MW-DCG-LCv-26 strain or a strain obtained after domestication of the *Clostridium ghonii;* and the *Clostridium ghonii* MW-DCG-LCv-26 is deposited in the National Measurement Institute, Australia, with a deposit number of V12/001486. Other preferred strains may be MW-DCG-HNCv-18 strain, deposited in the National Measurement Institute, Australia, with a deposit number of V12/001485; or an MW-DCG-CCv-17 strain, deposited in the National Measurement Institute, Australia, with a deposit number of V12/001487.

According the present disclosure, preferably, the *Clostridium ghonii* may be in a spore form.

Preferably, the spore form of the *Clostridium ghonii* may be freeze-dried powder with an auxiliary material of 1% sucrose.

Preferably, the pembrolizumab is a PD-1 antibody injection.

More preferably, a freeze-drying procedure of the *Clostridium ghonii* spore freeze-dried powder for injection includes: freezing at -40°C for 4 h, vacuumizing at -35°C for 10 min, and freeze-drying at -30°C for 10 min, -25°C for 10 min, -20°C for 26 h, -15°C for 2 h, -10°C for 10 min, -5°C for 10 min, 0°C for 10 min, 10°C for 2 h, 15°C for 10 min, 20°C for 3 h and 27°C for 3 h.

A preferred pharmaceutical combination of the present disclosure is 1×10⁷ CFU of the *Clostridium ghonii* spore freeze-dried powder combined with 0.2 mg of a pembrolizumab injection at a concentration of 1 mg/mL.

Further preferably, the pembrolizumab injection has a solvent of a sodium chloride injection with a mass percentage of 0.9%; and the *Clostridium ghonii* spore freeze-dried powder has a solvent of sterile water for injection and a sodium chloride injection with a mass percentage of 0.9%.

Further preferably, the *Clostridium ghonii* spore combined with pembrolizumab is used in a sequence of the *Clostridium ghonii* spore and then the pembrolizumab.

The present disclosure has the following beneficial effects:

It is found for the first time that the *Clostridium ghonii* spore combined with pembrolizumab can significantly improve a curative effect of colon cancer and reduce a dose of the pembrolizumab, and thus is efficient and low-toxic. Oncolysis by *Clostridium ghonii* can affect immunogenicity of a tumor microenvironment (TME) by various ways, converts an immunosuppressive state of the TME into an immune-activated state, adjusts the immunosuppressive TME, and breaks an immune tolerance. An optimal combination of the *Clostridium ghonii* spore and the pembrolizumab thoroughly removes about 20% of mouse tumor tissues. A benefit range of patients with tumors treated by a PD-1 antibody is expanded. The combination even has an obvious curative effect on patients failed the treatment by the PD-1 antibody.

### BRIEF DESCRIPTION OF THE DRAWINGS

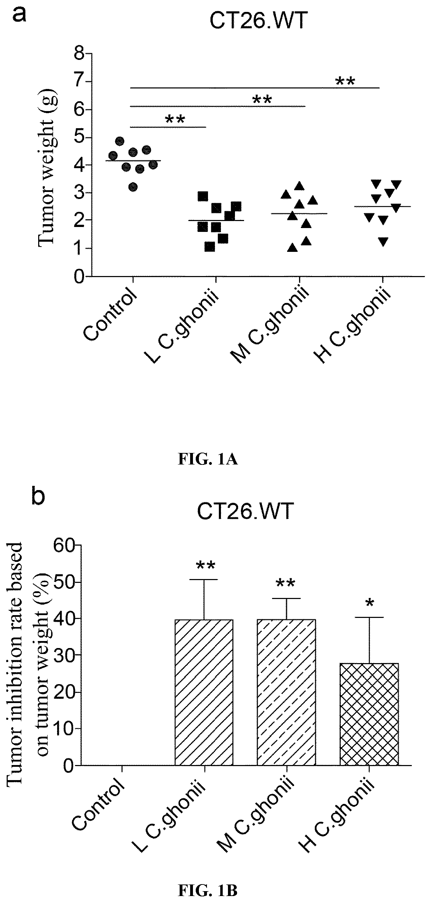

FIG. 1A and FIG. 1B respectively show tumor weight and a tumor inhibition rate based on tumor weight of each group in a CT26.WT colon cancer tumor-bearing mouse model treated with a *Clostridium ghonii* spore in Example 1; and data are expressed as: * indicates P *<* 0.05 compared with a Control group; and ** indicates P < 0.01 compared with the Control group;

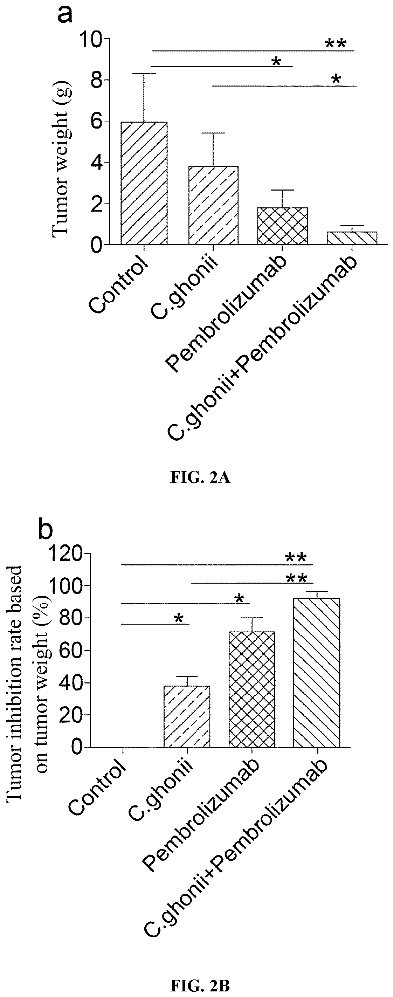

FIG. 2A and FIG. 2B respectively tumor weight and a tumor inhibition rate based on tumor weight of each group in a MC38 colon cancer tumor-bearing mouse model treated with a *Clostridium ghonii* spore combined with pembrolizumab in Example 2; and data are expressed as: * indicates P < 0.05; and ** indicates P < 0.01;

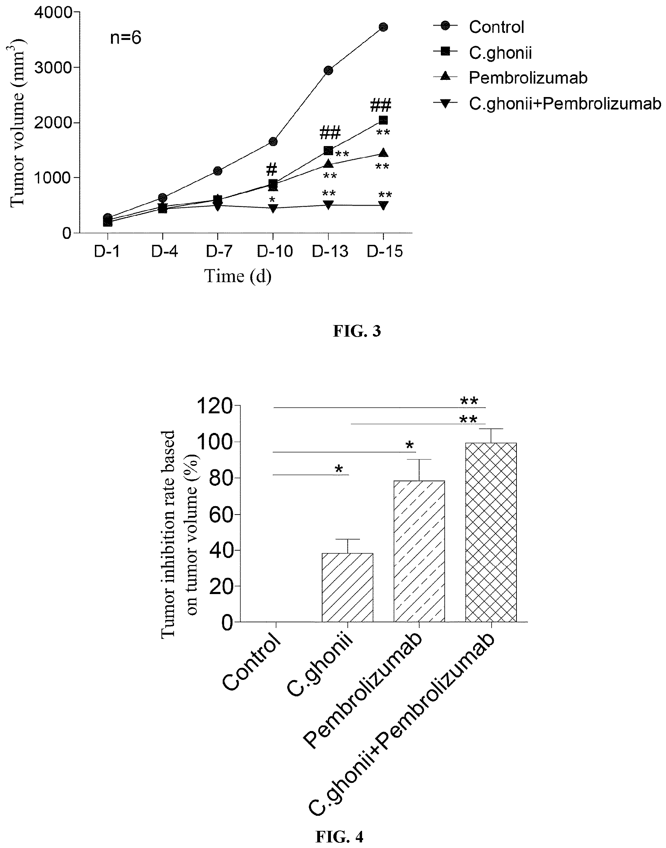

FIG. 3 shows a tumor volume change of each group in a MC38 colon cancer tumor-bearing mouse model treated with a *Clostridium ghonii* spore combined with pembrolizumab in Example 2; and data are expressed as: * indicates P < 0.05 compared with a Control group; ** indicates P < 0.01 compared with the Control group; ^{#} indicates P < 0.05 in a C. ghonii+Pembrolizumab group compared with a C. *ghonii* group; and ^{##} indicates P < 0.01 in the C. ghonii+Pembrolizumab group compared with the C. *ghonii* group;
FIG. 4 shows a tumor inhibition rate based on tumor volume at an experimental endpoint in a MC38 colon cancer tumor-bearing mouse model treated with a *Clostridium ghonii* spore combined with pembrolizumab in Example 2; and data are expressed as: * indicates P < 0.05; and ** indicates P < 0.01;

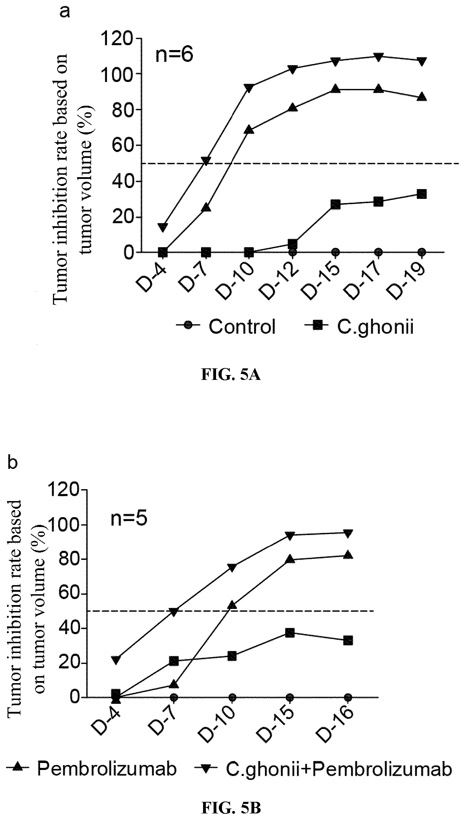

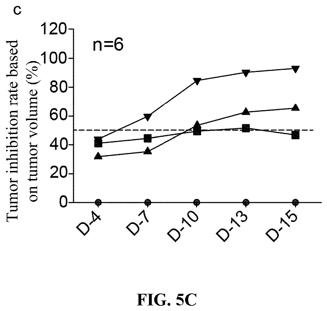

FIGS. 5A-5C shows a tumor inhibition rate based on tumor volume of each batch in a MC38 colon cancer tumor-bearing mouse model treated with a *Clostridium ghonii* spore combined with pembrolizumab in Example 2; specifically, FIG. 5A shows experiment batch 1, FIG. 5B shows experiment batch 2 and FIG. 5C shows experiment batch 3;

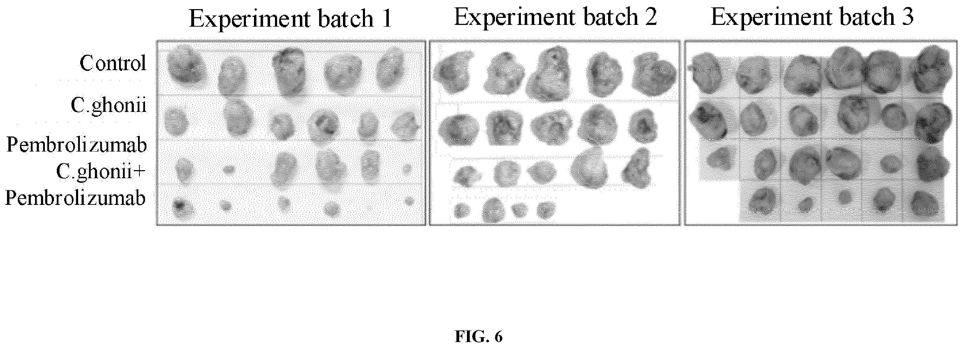

FIG. 6 shows morphological photos of tumors of each group in a MC38 colon cancer tumor-bearing mouse model treated with a *Clostridium ghonii* spore combined with pembrolizumab in Example 2;

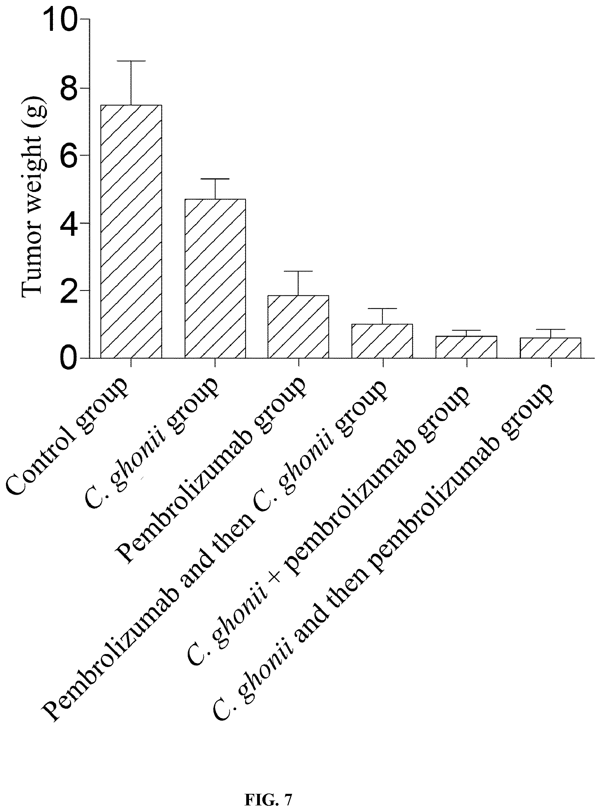

FIG. 7 shows tumor weight of each group in a MC38 colon cancer tumor-bearing mouse model treated with a *Clostridium ghonii* spore combined with pembrolizumab in a different administration sequence in Example 3; and

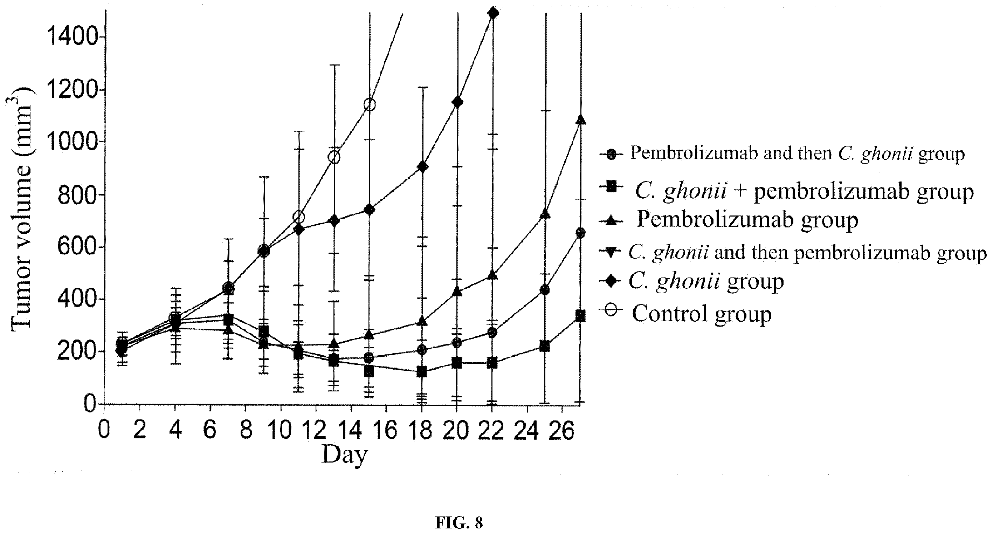

FIG. 8 shows a tumor volume change of each group in a MC38 colon cancer tumor-bearing mouse model treated with a *Clostridium ghonii* spore combined with pembrolizumab in a different administration sequence in Example 3.

### DETAILED DESCRIPTION OF THE EMBODIMENTS

The invention is defined in the claims.

The present disclosure will be further described in detail below in conjunction with specific examples. The described examples are only a part of technical solutions listed in the present disclosure for the convenience of the public to understand. These examples are only used to illustrate the present disclosure and not to limit the protection scope of the present disclosure. The protection scope of the present disclosure is defined by the claims.

### Example 1 Therapeutic effect of Clostridium ghonii spore on colon cancer tumor-bearing mouse model

Materials: *Clostridium ghonii* spore dried powder for injection: an MW-DCG-LCv-26 strain was deposited in the National Measurement Institute, Australia, with a deposit number of V12/001486 and developed by Shandong Xinchuang Biotechnology Co., Ltd. The *Clostridium ghonii* spore freeze-dried powder for injection used a *Clostridium ghonii* spore as an active ingredient and 1% sucrose as an auxiliary material. A freeze-drying procedure included: freezing at -40°C for 4 h, vacuumizing at -35°C for 10 min, and freeze-drying at -30°C for 10 min, -25°C for 10 min, -20°C for 26 h, -15°C for 2 h, -10°C for 10 min, -5°C for 10 min, 0°C for 10 min, 10°C for 2 h, 15°C for 10 min, 20°C for 3 h and 27°C for 3 h. The freeze-dried powder had a specification of 1×10⁸ CFU/bottle. A reference substance freeze-dried powder had a batch number: 201803001F, and was developed by Shandong Xinchuang Biotechnology Co., Ltd., by the above freeze-drying procedure with 1 mL of a 1% sucrose solution. A 0.9% sodium chloride injection had a batch number: 1803122161, and was commercially available in CISEN Pharmaceutical Co., LTD. Sterile water for injection had a batch number: 1704242163, and was commercially available in CISEN Pharmaceutical Co., LTD. CT26.WT colon cancer cells had a number of 3131C0001000800037 and were deposited in Cell Resource Center, Shanghai Institutes for Biological Sciences, Chinese Academy of Sciences. BALB/c female mice, with animal qualification certificate numbers: No.11401300088557 (120 mice), No.11401300089721 (60 mice), and No.11401300089721 (60 mice), were commercially available in Beijing HFK Bioscience Co., Ltd.

Methods: the CT26.WT cells were recovered and passaged to the required number, and prepared into a cell suspension for inoculation with a concentration of 7.5×10⁶ cells/mL and a cell viability over 90% to establish a subcutaneous xenograft model for colon cancer in the BALB/c mice. 0.2 mL of the cell suspension was subcutaneously inoculated on right forelimbs of the mice. Experimental animals with a tumor volume of about 0.30 cm³ were selected for an experiment in about 10 days. The tumor-formation conforming mice were randomly divided into 4 groups by lottery: control group (Control) , low-dose treatment group (L *C. ghonii),* medium-dose treatment group (M *C. ghonii),* and high-dose treatment group (H *C. ghonii),* with 8 mice in each group. The *Clostridium ghonii* spore dried powder was first re-dissolved with 0.1 mL of sterile water for injection and then prepared into suspensions with concentrations of 5×10⁷ cfu/mL, 1×10⁸ cfu/mL, and 2×10⁸ cfu/mL separately using a sodium chloride injection with a mass percentage of 0.9%. The mice were intratumorally injected with 0.1 mL of the suspensions separately once every other day for a total of 5 times at a dose of 5×10⁶ cfu/time, 1×10⁷ cfu/time, and 2×10⁷ cfu/time separately. The mice in the control group were injected with a reference substance at a concentration the same as that of the suspension in the high-dose treatment group. After the administration began, animal behaviors, death, near-death, and other clinical symptoms were observed every day. All surviving animals were dissected on the 2nd day after the last administration. Tumor weight was weighed and a tumor inhibition rate based on the tumor weight was calculated. Tumor inhibition rate based on tumor weight (IR_{TW}%)=(average tumor weight of control group-average tumor weight of experimental group)/average tumor weight of control group×100%. 3 batches of independent repeated experiments were conducted separately using the experimental method.

Results: during the experiment, all animals in 3 batches of the experiments did not show near-death/death. At the end of the experiments, the average tumor weight of the tumor-bearing mice in each group and the tumor inhibition rate in the treatment groups in 3 batches of the experiments were shown in Table 1 and Table 2 separately.

**Table 1 Average tumor weight of tumor-bearing mice in each group (g)**

| Experiment batch | Control group | L *C. ghonii* group | M *C. ghonii* group | H *C. ghonii* group |
|---|---|---|---|---|
| 1 | 2.85±0.59 | 1.98±0.76 | 1.84±0.67 | 2.45±0.34 |
| 2 | 4.15±0.51 | 2.00±0.61 | 2.23±0.78 | 2.50±0.71 |
| 3 | 2.69±0.52 | 1.73±0.56 | 1.72±0.42 | 1.93±0.39 |

**Table 2 Tumor inhibition rate based on tumor weight (%) in treatment groups**

| Experiment batch | L *C. ghonii* group | M *C. ghonii* group | H *C. ghonii* group |
|---|---|---|---|
| 1 | 30.40 | 35.58 | 14.12 |
| 2 | 51.90 | 46.17 | 39.79 |
| 3 | 35.46 | 35.97 | 28.06 |

The tumor weight of the mice in the treatment groups in each batch was separately smaller than that of mice in the Control group, and a best anti-tumor effect was shown in the M *C. ghonii* group (FIG. 1A). An inhibitory effect on tumor growth was shown in the treatment groups of each dose, a best tumor inhibition rate based on tumor weight was shown in the M C. *ghonii* group in experiment batches 1 and 3, and a best tumor inhibition rate based on tumor weight was shown in the L C. *ghonii* group in experimental batch 2. Statistical analysis based on the results of 3 batches of the experiments showed that the tumor inhibition rate based on tumor weight in the H C. *ghonii* group was significantly different from that in the Control group (P < 0.05) and the tumor inhibition rate based on tumor weight in the L C. *ghonii* group and the M C. *ghonii* group was separately extremely significant different from that in the Control group (both *P* < 0.01) as shown in FIG. 1B.

In conclusion, the *Clostridium ghonii* spore freeze-dried powder had an inhibitory effect on growth of colon cancer and the effect was better in the M C. *ghonii* group (1×10⁷ cfu/time) than in other treatment groups. Therefore, a preferred dose of the *C. ghonii* combined with pembrolizumab in a next experiment was 1×10⁷ cfu/time.

### Example 2 Therapeutic effect of Clostridium ghonii spore combined with pembrolizumab on PD-1 humanized transgenic colon cancer tumor-bearing mouse model

Materials: *Clostridium ghonii* spore dried powder for injection was developed by Shandong Xinchuang Biotechnology Co., Ltd., and a preparation method was the same as that in the Materials of Example 1; a pembrolizumab injection had a batch number of S001188 and a specification of 100 mg/4 mL, and was commercially available in Merck & Co., Inc.; reference substance freeze-dried powder had a batch number of 201910002F and 201803001F and was developed by Shandong Xinchuang Biotechnology Co., Ltd., and a preparation method was the same as that in the Materials of Example 1; a 0.9% sodium chloride injection had a batch number of 1809282161 and was commercially available in CISEN Pharmaceutical Co., LTD.; sterile water for injection had a batch number of 1902212162 and was commercially available in CISEN Pharmaceutical Co., LTD.; MC38 colon cancer cells had a Art.No. T1917 and were commercially available in Abm Biotechnology Co., Ltd.; and C57BL/6 PD-1 humanized genetically engineered mice, with an animal certificate number of No.20170010002500 (90 mice), No.312024300008757 (35 mice) and No.20170010001319 (50 mice), were commercially available in Shanghai Model Organisms.

Methods: the MC38 cells were recovered and passaged to the required number, and prepared into a cell suspension for inoculation with a concentration of 7.5×10⁶ cells/mL and a cell viability over 90% to establish a subcutaneous xenograft model for colon cancer in the C57BL/6 PD-1 humanized genetically engineered mice. 0.2 mL of the cell suspension was subcutaneously inoculated on right forelimbs of the mice. Experimental animals with a tumor volume larger than 0.15 cm³ were selected for an experiment in about 10 d. The screened conforming mice were randomly divided into 4 groups by lottery: control group (Control) , *Clostridium ghonii* spore treatment group *(C. ghonii),* pembrolizumab group, and *Clostridium ghonii* spore combined pembrolizumab group (C. Ghonii+Pembrolizumab), with more than or equal to 5 mice in each group. The *Clostridium ghonii* spore dried powder was first re-dissolved with 0.1 mL of sterile water for injection and then prepared into a suspension with a concentration 1×10⁸ cfu/mL using a sodium chloride injection with a mass percent of 0.9%. The mice were intratumorally injected with 0.1 mL of the suspension at a dose of 1×10⁷ cfu/time in the *C. ghonii* group and the C. Ghonii+Pembrolizumab group and at a dose of 0 cfu/time in the Control group and the Pembrolizumab group once every other day for a total of 6 times. The pembrolizumab injection was diluted into a 1 mg/mL working solution using the 0.9% sodium chloride injection. The mice were intraperitoneally injected with 0.2 mL of the working solution in the Pembrolizumab group and the C. Ghonii+Pembrolizumab group at 0.2 mg/time and intraperitoneally injected with 0.2 mL of the 0.9% sodium chloride injection twice every week for a total of 4 times.

The mice were firstly administrated with the *Clostridium ghonii* spore and then a PD-1 antibody in the following administration sequence: *Clostridium ghonii* spore was injected every other day from the 1st day; and pembrolizumab was injected on the 3rd, 6th, 9th and 13rd day separately.

Observation and evaluation indicators: after the administration began, animal behaviors, death or near-death were observed every day. A tumor volume was observed every other 1-2 d and a tumor inhibition rate was calculated based on the tumor volume measured at a last time of the experiment. All surviving animals were dissected on the 2nd day after the last administration. Tumor weight was weighed and a tumor inhibition rate based on the tumor weight was calculated. Tumor inhibition rate based on tumor weight (IRTW%)=(average tumor weight of control group-average tumor weight of experimental group)/average tumor weight of control group×100%. Tumor inhibition rate based on tumor volume (IRTW%)=1-(average tumor volume of experimental group at a beginning-average tumor volume of experimental group at an end)/(average tumor volume of control group at a beginning-average tumor volume of control group at an end)×100%. Cure rate (%) = number of cured animals in each group/total number of experimental animals in each group×100% (at an end of the experiment).

3 batches of independent repeated experiments were conducted separately using the experimental method.

Results: during the experiment, 1 mouse in the control group of experiment batch 1 was dead, but mice in other batches or other groups did not show death or near-death.

### 1. Average tumor weight and tumor inhibition rate of tumor-bearing mice in each group

The average tumor weight of the tumor-bearing mice in each group in 3 batches of the experiments was shown in Table 3 separately. The average tumor weight of the mice in each treatment group was smaller than that of the mice in the Control group, and the tumor weight of the mice in the C. ghonii+Pembrolizumab group was smaller than that of the mice in any single drug treatment group, and was extremely significant lower than that of mice in the Control group *(P* < 0.01). The average tumor weight of the mice in the C. ghonii+Pembrolizumab group was also significantly smaller than that of mice in the single C. *ghonii* group and the single Pembrolizumab group separately (both P < 0.05) (FIG. 2A).

**Table 3 Average tumor weight of tumor-bearing mice in each group (g)**

| Experiment batch | Control group | *C. ghonii* group | Pembrolizumab group | C. ghonii+Pembrolizumab group |
|---|---|---|---|---|
| 1 | 3.46±1.33 | 2.24±0.74 | 0.83±0.74 | 0.18±0.19 |
| 2 | 7.97±2.46 | 5.42±1.70 | 1.92±1.42 | 0.56±0.45 |
| 3 | 6.41±1.46 | 3.60±1.09 | 2.48±1.40 | 0.86±0.79 |

Statistical analysis based on the results of 3 batches of the experiments showed that an inhibitory effect on tumor growth was exhibited in each treatment group with a tumor inhibition rate above 30%. The tumor inhibition rate: C. ghonii+Pembrolizumab group > Pembrolizumab group > C. *ghonii* group (FIG. 2B). After administration of the pembrolizumab at a same dose, the tumor inhibition rate in the C. ghonii+Pembrolizumab group increased by approximately 20%, 20%, and 25% separately compared with that in the single Pembrolizumab group (Table 4).

**Table 4 Tumor inhibition rate based on tumor weight (%) in treatment groups**

| Experiment batch | *C. ghonii* group | Pembrolizumab group | C. ghonii+Pembrolizumab group |
|---|---|---|---|
| 1 | 35.33 | 76.04 | 94.76 |
| 2 | 32.03 | 75.95 | 92.95 |
| 3 | 43.82 | 61.27 | 86.61 |

### 2. Average tumor volume and tumor inhibition rate of tumor-bearing mice in each group

Before the experiment began, there was no significant difference in the average tumor volume of mice in each group of each batch (all *P* > 0.05). At an end of administration of the tumor-bearing mice in experiment batch 3, the tumor volume of mice in the C. *ghonii* group, the Pembrolizumab group, and the C. ghonii+Pembrolizumab group was significantly smaller than that of mice in the Control group separately (P = 0.001, *P* = 0.000053, and P = 0.000). The tumor volume of the mice in the C. ghonii+Pembrolizumab group was obviously smaller than that of mice in the C. *ghonii* group and the Pembrolizumab group. Besides, on the 10th day, there was a significant difference in the average tumor volume between the C. ghonii+Pembrolizumab group and the Control group. Meanwhile, the tumor volume of the mice in the C. ghonii+Pembrolizumab group was nearly 1 time smaller than that of the mice in the Pembrolizumab group (FIG. 3).

The tumor inhibition rate was calculated based on the tumor volume. During 3 batches of the experiments, the tumor inhibition rate in the C. ghonii+Pembrolizumab group was higher than that in the other groups (FIG. 4).

On the 7th day after administration in 3 batches of the experiments, the pembrolizumab was administered twice. After the pembrolizumab was administered at a dose of 0.4 mg, the tumor inhibition rate in the C. ghonii+Pembrolizumab group was 51.29% (greater than 50%), 49.78% (approximately 50%), and 59.33% (greater than 50%) separately, while the tumor inhibition rate in the single Pembrolizumab group was 25.67%, 7.68%, and 35.10% separately. After the mice in the single Pembrolizumab alone group were administered 3 times with pembrolizumab at a total dose of 0.6 mg, the tumor inhibition rate reached that of the C. ghonii+Pembrolizumab group when the pembrolizumab was administered twice (at a total dose of 0.4 mg), and were 69.31%, 52.83%, and 53.08% separately (FIGS. 5A-5C). It can be seen that an anti-tumor effect of the pembrolizumab was significantly improved in the C. ghonii+Pembrolizumab group. The dose of the pembrolizumab required to obtain a same therapeutic effect in the C. ghonii+Pembrolizumab group was reduced by 50%, thus high efficiency and low toxicity were reached.

### 3. Cure rate

About 20% (1/6, 1/5, and 1/6) of mouse tumors were completely eliminated in 3 batches of the experiments in the C. ghonii+Pembrolizumab group (Table 5), and no tumor growth was seen at the end of the experiments. But no complete tumor elimination was shown in the Pembrolizumab group and the C. *ghonii* group, that is, the cure rate was 0%. This may due to the fact that a *Clostridium ghonii* spore germinated in a hypoxic area of a tumor, could affect immunogenicity of a tumor microenvironment (TME) by various ways, converted an immunosuppressive state of the TME into an immune-activated state, adjusted the immunosuppressive TME, and broke an immune tolerance. Combined with the pembrolizumab, a therapeutic effect of the PD-1 antibody can be further enhanced, and about 20% of the mice were cured. The *Clostridium ghonii* was expected to become an excellent "sensitizer" for immunotherapy of a patient with a tumor. Morphology of tumors of each group was shown in FIG. 6.

**Table 5 Cure rate (%)**

| Experiment batch | Control group | *C. ghonii* group | Pembrolizumab group | C. ghonii+Pembrolizumab group |
|---|---|---|---|---|
| 1 | 0 | 0 | 0 | 16.7 |
| 2 | 0 | 0 | 0 | 20.0 |
| 3 | 0 | 0 | 0 | 16.7 |

### Example 3 Influence of administration sequence of Clostridium ghonii spore and pembrolizumab on therapeutic effect on PD-1 humanized transgenic colon cancer tumor-bearing mouse model

Materials: *Clostridium ghonii* spore dried powder for injection was developed by Shandong Xinchuang Biotechnology Co., Ltd., and a preparation method was the same as that in the Materials of Example 1; a pembrolizumab injection had a batch number of S006648 and a specification of 100 mg/4 mL, and was commercially available in Merck & Co., Inc.; reference substance freeze-dried powder had a batch number of 201910002F and was developed by Shandong Xinchuang Biotechnology Co., Ltd., and a preparation method was the same as that in the Materials of Example 1; a 0.9% sodium chloride injection had a batch number of J18070104 and was commercially available in Shandong Hualu Pharmaceutical Co. Ltd.; sterile water for injection had a batch number of 1902212162 and was commercially available in CISEN Pharmaceutical Co., LTD.; MC38 colon cancer cells had a Art.No. T1917 and were commercially available in Abm Biotechnology Co., Ltd.; and C57BL/6 PD-1 humanized genetically engineered mice, with an animal certificate number of No.20170010002500 (90 mice), were commercially available in Shanghai Model Organisms.

Methods: A subcutaneous xenograft model for MC38 colon cancer in the C57BL/6 PD-1 humanized genetically engineered mice was established with a same method as Example 2. The successfully modeled mice were randomly divided by lottery into A: control group (Control), B: *Clostridium ghonii* spore group *(C. ghonii),* C: Pembrolizumab group, D: first Pembrolizumab and then C. *ghonii* group, E: first C. *ghonii* and then Pembrolizumab group, F: C. ghonii+Pembrolizumab simultaneous group, 8 mice in each group; the C. *ghonii* was administrated at a dose of 0 cfu/time, 1×10⁷ cfu/time, 0 cfu/time, 1×10⁷ cfu/time, 1×10⁷ cfu/time and 1×10⁷ cfu/time correspondingly, with an administration volume of 0.1 mL/time, once every other day; and 0.2 mL of 1 mg/mL of a Pembrolizumab injection was administered intraperitoneally, at a dose of 0.2 mg/time, once every other day. An administration process in a first stage was shown in Table 6.

**Table 6 Administration process in first stage**

| Groupi ng | First administration | | Second administration | | Third administration | | Last administration | |
|---|---|---|---|---|---|---|---|---|
| | C. *ghon ii* | Pembrolizu mab | C. *ghon ii* | Pembrolizu mab | *C. ghon ii* | Pembrolizu mab | C. *ghon ii* | Pembrolizu mab |
| A | | | | | | | | |
| B | √ | | √ | | √ | | √ | |
| C | | √ | | √ | | √ | | √ |
| D | | √ | √ | √ | √ | √ | | √ |
| E | √ | | √ | √ | √ | √ | √ | |
| F | √ | √ | √ | √ | √ | √ | √ | √ |

After the administration in the first stage, the mice were observed for 6 days. The administration process was repeated in a second stage as in the first stage.

Observation and evaluation indicators: after the administration began, animal behaviors, death or near-death were observed every day. A tumor volume was observed every other 1-2 d and a tumor inhibition rate was calculated based on the tumor volume measured at a last time of the experiment. All surviving animals were dissected on the 8th day after the last administration of the second stage. Tumor weight was weighed and a tumor inhibition rate based on the tumor weight was calculated.Tumor inhibition rate based on tumor weight (IRTW%)=(average tumor weight of control group-average tumor weight of experimental group)/average tumor weight of control group×100%.Cure rate (%) = number of cured animals in each group/total number of experimental animals in each group × 100% (at an end of the experiment).

Results: during the experiment, 4 mouse in the Control group were dead due to oversized or ruptured tumors, but mice in other groups did not show death or near-death.

### 1. Tumor weight and tumor inhibition rate

Compared with the Control group, the tumor weight in the C. *ghonii* group, the Pembrolizumab group, the first Pembrolizumab and then C. *ghonii* group, the first C. *ghonii* and then Pembrolizumab group, and the C. ghonii+Pembrolizumab simultaneous group was separately significantly smaller than that in the Control group (P = 0.005, *P* = 0.000, *P* = 0.000, *P* = 0.000, and P = 0.000). The tumor weight of mice in each group was shown Table 7 and FIG. 7.

**Table 7 Tumor weight**

| Groupin g | Control group | C. *ghonii* group | Pembrolizum ab group | First Pembrolizum ab and then *C. ghonii* group | First C. *ghonii* and then Pembrolizum ab group | *C. ghonii+* Pembrolizum ab group |
|---|---|---|---|---|---|---|
| Tumor weight (g) | 7.43±2.6 6 | 4.65±1.7 2 | 1.77±2.08** | 0.95±1.29** | 0.54±0.69** | 0.58±0.56** |

| | | | | | | |
|---|---|---|---|---|---|---|
| Note: ** indicates P < 0.01 compared with the Control group. | | | | | | |

The tumor inhibition rate was calculated according to tumor weight. The results showed that the tumor inhibition rate in the C. *ghonii* group, the Pembrolizumab group, the first Pembrolizumab and then C. *ghonii* group, the first C. *ghonii* and then Pembrolizumab group, and the C. ghonii+Pembrolizumab group was 37.39%, 76.20%, 87.26%, 92.71%, and 92.19% separately. A significant inhibitory effect on tumor growth was shown in each treatment group (Table 8).

**Table 8 Tumor inhibition rate**

| Grouping | C. *ghonii* group | Pembrolizumab group | First Pembrolizumab and then C. *ghonii* group | First C. *ghonii* and then Pembrolizumab group | *C. ghonii* +Pembrolizumab group |
|---|---|---|---|---|---|
| Tumor inhibition rate (%) | 37.39 | 76.20 | 87.26 | 92.71 | 92.19 |

### 2. Tumor volume and tumor inhibition rate

Before the treatment, there was no significant difference in the tumor volume of the tumor-bearing mice in each group (all *P* > 0.05). At the end of the administration, the tumor volume of the mice in the C. *ghonii* group, the Pembrolizumab group, the first Pembrolizumab and then *C. ghonii* group, the first C. *ghonii* and then Pembrolizumab group, and C. ghonii+Pembrolizumab simultaneous group was separately significantly smaller than that of the mice in the Control group (P = 0.002, *P* = 0.000, *P* = 0.000, *P* = 0.000, and P = 0.000, FIG. 8). The tumor inhibition rate: first C. *ghonii* and then Pembrolizumab group > C. ghonii+Pembrolizumab simultaneous group > first Pembrolizumab and then C. *ghonii* group > Pembrolizumab group > Control group as shown in Table 9.

**Table 9 Tumor inhibition rate**

| Groupin g | Contro l group | C. *ghoni i* group | Pembrolizuma b group | First Pembrolizuma b and then C. *ghonii* group | First C. *ghonii* and then Pembrolizuma b group | *C. ghonii+* Pembrolizuma b group |
|---|---|---|---|---|---|---|
| Tumor inhibitio n rate (%) | --- | 40.99 | 79.07 | 87.36 | 93.62 | 93.42 |

### 3. Cure rate

During the experiment, the cure rate of the mice in each group was shown in Table 10.

**Table 10 Cure rate**

| Groupin g | Contro l group | C. *ghoni i* group | Pembrolizuma b group | First Pembrolizuma b and then C. *ghonii* group | First C. *ghonii* and then Pembrolizuma b group | *C. ghonii+* Pembrolizuma b group |
|---|---|---|---|---|---|---|
| cure rate (%) | 0 | 0 | 0 | 12.5 | 25 | 12.5 |

The tumor cure rate of the mice in the first C. *ghonii* and then Pembrolizumab group was significantly higher than that of the mice in other groups, and was twice the cure rate of the first Pembrolizumab and then C. *ghonii* group and C. ghonii+Pembrolizumab simultaneous group. This may be due to the fact that intratumoral administration of the *Clostridium ghonii* spore was effective in lysing tumor tissue without distinguishment and destroying the TME. At the same time, oncolysis by the bacteria could recruit immune cells to infiltrate into the TME, and changed the infiltration degree and the composition of the immune cells in the TME. A cell lysate would attract a large number of CD8⁺ T cells to aggregate. The PD-1 antibody treatment was conducted after the oncolysis by the bacteria, avoids immunosuppression in immunotherapy, launched a large-scale attack of T cells on tumor cells, and thus plays a "double" anti-cancer effect. Therefore, the administration sequence was also critical to the therapeutic effect on the tumors when the oncolysis by the bacteria combined with the immunosuppressive agent.

In conclusion, the first *Clostridium ghonii* spore treatment then combined with the immunotherapy could enhance an anti-tumor effect, reduced a dose of the immune drug, and thus was efficient and low-toxic.

Although the above example has described the present disclosure in detail, it is only a part of, not all of, the examples of the present disclosure. Other examples may also be obtained by persons based on the example without creative efforts, and all of these examples shall fall within the protection scope of the present disclosure.

## Claims

1. A *Clostridium ghonii* spore combined with pembrolizumab for use in the treatment of colon cancer.

2. The *Clostridium ghonii* spore combined with pembrolizumab for use according to claim 1, wherein the *Clostridium ghonii* spore is used before the pembrolizumab.

3. The *Clostridium ghonii* spore combined with pembrolizumab for use according to claim 2, wherein the *Clostridium ghonii* spore is a *Clostridium ghonii* spore freeze-dried powder for injection.

4. A drug for treating colon cancer, comprising active ingredients of a *Clostridium ghonii* spore and pembrolizumab.

5. The drug according to claim 4, wherein the *Clostridium ghonii* is a *Clostridium ghonii* MW-DCG-LCv-26 strain or a strain obtained after domestication of the *Clostridium ghonii;* and the *Clostridium ghonii* MW-DCG-LCv-26 strain is deposited in the National Measurement Institute, Australia, with a deposit number of V12/001486.

6. The drug according to claim 4, wherein the strain obtained after domestication of the *Clostridium ghonii* is an MW-DCG-HNCv-18 strain deposited in the National Measurement Institute, Australia, with a deposit number of V12/001485.

7. The drug according to claim 4, wherein the strain obtained after domestication of the *Clostridium ghonii* is an MW-DCG-CCv-17 strain deposited in the National Measurement Institute, Australia, with a deposit number of V12/001487.

8. The drug according to claim 4, wherein in the drug, the *Clostridium ghonii* spore is a *Clostridium ghonii* spore freeze-dried powder for injection with an auxiliary material of 1% sucrose.

9. The drug according to claim 8, wherein a freeze-drying procedure of the *Clostridium ghonii* spore freeze-dried powder for injection comprises: maintaining -40°C for 4 h, vacuumizing, and freeze-drying at -35°C for 10 min, -30°C for 10 min, -25°C for 10 min, -20°C for 26 h, -15°C for 2 h, -10°C for 10 min, -5°C for 10 min, 0°C for 10 min, 10°C for 2 h, 15°C for 10 min, 20°C for 3 h and 27°C for 3 h.

10. The drug according to claim 4, wherein the pembrolizumab is a PD-1 antibody injection.

11. The drug according to claim 4, wherein every 1×10⁷ CFU of the *Clostridium ghonii* spore is combined with 0.2 mg of a pembrolizumab solution at a concentration of 1 mg/mL.

12. The drug according to claim 11, wherein the pembrolizumab solution has a solvent of a sodium chloride injection with a mass percentage of 0.9%.

13. The drug according to claim 11, wherein the *Clostridium ghonii* spore is the *Clostridium ghonii* spore freeze-dried powder for injection, and the *Clostridium ghonii* spore freeze-dried powder for injection has a solvent of sterile water for injection and a sodium chloride injection with a mass percentage of 0.9%.

14. The drug according to any one of claims 4 to 13 for use in the treatment of colon cancer.

15. The drug for use according to claim 14, wherein the *Clostridium ghonii* spore is administered before the pembrolizumab.

## Patentansprüche

1. Eine *Clostridium ghonii* Spore kombiniert mit Pembrolizumab zur Verwendung in der Behandlung von Darmkrebs.

2. Die *Clostridium ghonii* Spore kombiniert mit Pembrolizumab zur Verwendung nach Anspruch 1, wobei die *Clostridium ghonii* Spore vor dem Pembrolizumab verwendet wird.

3. Die *Clostridium ghonii* Spore kombiniert mit Pembrolizumab zur Verwendung nach Anspruch 2, wobei die *Clostridium ghonii* Spore ein gefriergetrocknetes Pulver von *Clostridium ghonii* Sporen zur Injektion ist.

4. Ein Medikament zur Behandlung von Darmkrebs, das aktive Bestandsteile von einer *Clostridium ghonii* Spore und Pembrolizumab umfasst.

5. Das Medikament nach Anspruch 4, wobei das *Clostridium ghonii* ein *Clostridium ghonii* MW-DCG-LCv-26 Stamm oder ein Stamm, der nach der *Clostridium ghonii* Domestizierung gewonnen wurde, ist; und der *Clostridium ghonii* MW-DCG-LCv-26 Stamm in dem National Measurement Institute, Australien, mit der Hinterlegungsnummer V12/001486 hinterlegt ist.

6. Das Medikament nach Anspruch 4, wobei der Stamm, der nach der *Clostridium ghonii* Domestizierung gewonnen wurde, ein MW-DCG-HNCv-18 Stamm ist, der in dem National Measurement Institute, Australien, mit der Hinterlegungsnummer V12/001485 hinterlegt ist.

7. Das Medikament nach Anspruch 4, wobei der Stamm, der nach der *Clostridium ghonii* Domestizierung gewonnen wurde, ein MW-DCG-CCv-17 Stamm ist, der in dem National Measurement Institute, Australien, mit der Hinterlegungsnummer V12/001487 hinterlegt ist.

8. Das Medikament nach Anspruch 4, wobei in dem Medikament die *Clostridium ghonii* Spore ein gefriergetrocknetes Pulver von *Clostridium ghonii* Sporen zur Injektion mit einem Hilfsstoff von 1% Saccharose ist.

9. Das Medikament nach Anspruch 8, wobei ein Gefriertrocknungsverfahren des gefriergetrockneten Pulvers der *Clostridium ghonii* Spore zur Injektion umfasst: Halten bei -40°C für 4 h, Anlegen von Vakuum, und Gefriertrocknen bei -35°C für 10 min, -30°C für 10 min, -25°C für 10 min, -20°C für 26 h, -15°C für 2 h, -10°C für 10 min, -5°C für 10 min, 0°C für 10 min, 10°C für 2 h, 15°C für 10 min, 20°C für 3 h und 27°C für 3 h.

10. Das Medikament nach Anspruch 4, wobei das Pembrolizumab eine PD-1 Antikörper Injektion ist.

11. Das Medikament nach Anspruch 4, wobei jede 1×10⁷ KBE der *Clostridium ghonii* Spore mit 0.2 mg einer Pembrolizumab Lösung von einer Konzentration von 1 mg/mL kombiniert wird.

12. Das Medikament nach Anspruch 11, wobei die Pembrolizumab Lösung ein Lösungsmittel aus einer Natriumchlorid-Injektion mit einem Massenanteil von 0,9 % hat.

13. Das Medikament nach Anspruch 11, wobei die *Clostridium ghonii* Spore ein gefriergetrocknetes Pulver von *Clostridium ghonii* Spore zur Injektion ist und des gefriergetrocknete Pulver von *Clostridium ghonii* Sporen zur Injektion ein Lösungsmittel aus sterilem Wasser zur Injektion und einer Natriumchlorid-Injektion mit einem Massenanteil von 0,9 % hat.

14. Das Medikament nach irgendeinem der Ansprüche 4 bis 13 zur Verwendung bei der Behandlung von Darmkrebs.

15. Das Medikament zur Verwendung nach Anspruch 14, wobei die *Clostridium ghonii* Spore vor dem Pembrolizumab verabreicht wird.

## Revendications

1. Spore de *Clostridium ghonii* en combinaison avec pembrolizumab destinée à être utilisée dans le traitement du cancer du côlon.

2. Spore de *Clostridium ghonii* en combinaison avec pembrolizumab destinée à être utilisée selon la revendication 1, dans laquelle la spore de *Clostridium ghonii* est utilisée avant le pembrolizumab.

3. Spore de *Clostridium ghonii* en combinaison avec pembrolizumab destinée à être utilisée selon la revendication 2, dans laquelle la spore de *Clostridium ghonii* est une poudre de spores de *Clostridium ghonii* lyophilisée destinée à l'injection.

4. Médicament permettant de traiter le cancer du côlon, comprenant des principes actifs d'une spore de *Clostridium ghonii* et du pembrolizumab.

5. Médicament selon la revendication 4, dans lequel le *Clostridium ghonii* est une souche de *Clostridium ghonii* MW-DCG-LCv-26 ou une souche obtenue après domestication du *Clostridium ghonii* ; et la souche de *Clostridium ghonii* MW-DCG-LCv-26 est déposée au National Measurement Institute, Australie, sous le numéro de dépôt V12/001486.

6. Médicament selon la revendication 4, dans lequel la souche obtenue après domestication du *Clostridium ghonii* est une souche MW-DCG-HNCv-18 déposée au National Measurement Institute, Australie, sous le numéro de dépôt V12/001485.

7. Médicament selon la revendication 4, dans lequel la souche obtenue après domestication de *Clostridium ghonii* est une souche MW-DCG-CCv-17 déposée au National Measurement Institute, Australie, sous le numéro de dépôt V12/001487.

8. Médicament selon la revendication 4, dans lequel dans le médicament, la spore de *Clostridium ghonii* est une poudre de spores de *Clostridium ghonii* lyophilisée destinée à l'injection avec une matière auxiliaire contenant 1 % de saccharose.

9. Médicament selon la revendication 8, dans lequel la procédure de lyophilisation de la poudre de spores de *Clostridium ghonii* lyophilisée destinée à l'injection comprend : le maintien à -40°C pendant 4 h, la mise sous vide et la lyophilisation à -35°C pendant 10 min, -30°C pendant 10 min, -25°C pendant 10 min, -20°C pendant 26 h, -15°C pendant 2 h, -10°C pendant 10 min, -5°C pendant 10 min, 0°C pendant 10 min, 10°C pendant 2 h, 15°C pendant 10 min, 20°C pendant 3 h et 27°C pendant 3 h.

10. Médicament selon la revendication 4, dans lequel le pembrolizumab est une injection d'anticorps qui se lie au récepteur PD-1.

11. Médicament selon la revendication 4, dans lequel chaque 1×10⁷ UFC de spore de *Clostridium ghonii* est combinée à 0,2 mg d'une solution de pembrolizumab à une concentration de 1 mg/mL.

12. Médicament selon la revendication 11, dans lequel la solution de pembrolizumab a pour solvant une injection de chlorure de sodium avec un pourcentage en masse de 0,9 %.

13. Médicament selon la revendication 11, dans lequel la spore de *Clostridium ghonii* est la poudre de spores de *Clostridium ghonii* lyophilisée destinée à l'injection, et la poudre de spores de *Clostridium ghonii* lyophilisée destinée à l'injection a pour solvant de l'eau stérile pour injection et une injection de chlorure de sodium avec un pourcentage en masse de 0,9 %.

14. Médicament selon l'une quelconque des revendications 4 à 13 destiné à être utilisé dans le traitement du cancer du côlon.

15. Médicament selon la revendication 14, dans lequel la spore de *Clostridium ghonii* est administrée avant le pembrolizumab.
